(19) Europäisches Patentamt
European Patent Office
Office européen des brevets

(11) Veröffentlichungsnummer: **0 217 736 B1**

(12) **EUROPÄISCHE PATENTSCHRIFT**

(45) Veröffentlichungstag der Patentschrift: **15.04.92**

(51) Int. Cl.5: **C07D 457/12**, A61K 31/48

(21) Anmeldenummer: **86730153.3**

(22) Anmeldetag: **03.10.86**

Die Akte enthält technische Angaben, die nach dem Eingang der Anmeldung eingereicht wurden und die nicht in dieser Patentschrift enthalten sind.

# (54) 1,2-Disubstituierte Ergolinderivate.

(30) Priorität: **04.10.85 DE 3535929**

(43) Veröffentlichungstag der Anmeldung:
**08.04.87 Patentblatt 87/15**

(45) Bekanntmachung des Hinweises auf die Patenterteilung:
**15.04.92 Patentblatt 92/16**

(84) Benannte Vertragsstaaten:
**AT BE CH DE ES FR GB GR IT LI LU NL SE**

(56) Entgegenhaltungen:
**EP-A- 0 056 358**
**EP-A- 0 082 808**
**EP-A- 0 118 848**
**EP-A- 0 160 842**

**COLLECTION CZECHOSLOVAK CHEM. COMMUN., Band 47, 1982, Seiten 1757-1761, Prague, CS; J. BENES et al.: "2-Acylated derivatives of ergoline"**

**CHEMICAL ABSTRACTS, Band 105, 1986, Seite 674, Ref.Nr. 79217u, Columbus, Ohio, US & CS-A-221 421 (J. BENES et al.) 15-02-1986**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Huth, Andreas, Dr.**
**Kirchweg 55**
**W-1000 Berlin 38(DE)**
Erfinder: **Sauer, Gerhard, Dr.**
**Königsbacher Zeile**
**W-1000 Berlin 28(DE)**
Erfinder: **Wachtel, Helmut, Dr.**
**Asternplatz 2**
**W-1000 Berlin 45(DE)**

**EP 0 217 736 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 1,2-disubstituierte-Ergolinderivate, ihre Herstellung und ihre Verwendung als Arzneimittel.

Die erfindungsgemäßen Verbindungen haben die allgemeine Formel I

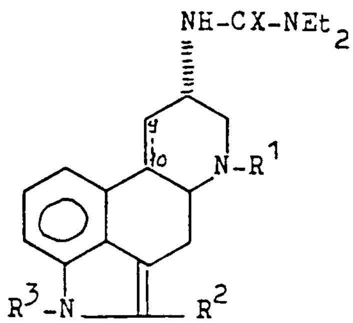

(I),

worin

X ein Sauerstoff- oder Schwefelatom bedeutet,

$R^1$ eine $C_{1-6}$-Alkylgruppe ist

$R^2$ Halogen, eine gesättigte oder ungesättigte $C_{1-6}$-Alkylgruppe, die gegebenenfalls substituiert sein kann mit $OR^4$ oder einem gegebenenfalls mit $C_{1-2}$-Alkyl, $C_{1-2}$-Alkoxy oder Halogen substituierten Phenyl-, Pyridin-, Thiophen-, Furan-, Pyrimidin-, Imidazol- oder Pyrazol-Rest, wobei $R^4$ Wasserstoff, $C_{1-6}$-Alkyl, Tetrahydropyranyl oder $C_{5-6}$-Cycloalkyl bedeutet,
eine $S$-$R^5$-Gruppe, worin $R^5$ eine $C_{1-6}$-Alkylgruppe, die gegebenenfalls mit Phenyl substituiert sein kann oder einen gegebenenfalls mit $C_{1-2}$-Alkyl, $C_{1-2}$-Alkoxy oder Halogen substituierten Phenyl-, Pyridin-, Thiophen-, Furan-, Pyrimidin-, Imidazol- oder Pyrazol-Rest darstellt,
eine

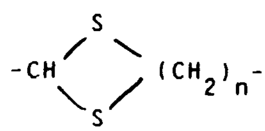

Gruppe, worin n 2 oder 3 ist oder
eine CHO-Gruppe bedeutet,

$R^3$ $C_{1-6}$-Alkyl oder $C_{2-4}$-Acyl ist und

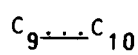

eine CC-Einfach- oder eine CC-Doppelbindung bedeutet und das Wasserstoffatom in 10-Stellung α-ständig ist, wenn

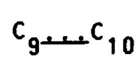

eine CC-Einfachbindung darstellt, sowie deren Säureadditionssalze.

Unter niederen Alkylresten versteht man solche mit bis zu 6 Kohlenstoffatomen, wobei $C_1$-$C_4$-Alkyle bevorzugt sind wie zum Beispiel Methyl, Ethyl, Isopropyl, n-Propyl, n-Butyl, Isobutyl und tert. Butyl.

Ist der niedere Alkylrest ungesättigt, so sind niedere Alkenyl- oder Alkinylreste gemeint, die bevorzugt 2 - 3 Kohlenstoffatome haben und mit den genannten Resten substituiert sein können.

Acylreste leiten sich von aliphatischen Carbonsäuren mit bevorzugt 2 - 4 Kohlenstoffatomen ab wie beispielsweise Essigsäure, Propionsäure und Buttersäure.

Als Halogene $R^2$ sind bevorzugt Chlor, Brom oder Jod geeignet.

Der aromatische Rest in $R^2$ enthält bis zu 6 Kohlenstoffatome, wobei ein oder mehrere Kohlenstoffatome durch Heteroatome wie Sauerstoff, Schwefel oder Stickstoff ersetzt sein können. Beispielsweise kommen als aromatische und heteroaromatische Reste in Frage: Phenyl, Pyridinyl, Thiophenyl, Furanyl, Pyrimidinyl, Imidazolyl, Pyrazolyl u.a.

Der Aromat kann in jeder beliebigen Position ein- oder mehrfach substituiert sein, beispielsweise mit niederem Alkyl, niederem Alkoxy, Halogen wie Fluor, Chlor, Brom oder Jod und anderen Substituenten.

Niedere Alkylgruppen im aromatischen Rest haben insbesondere 1 - 2 Kohlenstoffatome.

Als bevorzugte Aralkylreste sind solche mit bis zu 2 Kohlenstoffatomen im Alkylrest anzusehen wie beispielsweise der Benzyl- oder Phenethylrest. Als Substituenten sind die oben genannten aromatischen Substituenten geeignet.

Der Cycloalkylrest hat bevorzugt 5- oder 6-Kohlenstoffatome wie beispielsweise Cyclopentyl oder Cyclohexyl.

Die Salze der erfindungsgemäßen Verbindungen der Formel I sind Säureadditionssalze und leiten sich von üblicherweise verwendeten Säuren ab. Solche Säuren sind zum Beispiel anorganische Säuren, wie beispielsweise Chlorwasserstoffsäure, Salpetersäure, Phosphorsäure. Schwefelsäure, Bromwasserstoffsäure, Jodwasserstoffsäure, salpetrige Säure oder phosphorige Säure, oder organische Säuren, wie beispielsweise aliphatische Mono- oder Dicarbonsäuren, phenylsubstituierte Alkancarbonsäuren, Hydroxyalkancarbonsäuren oder Alkendicarbonsäuren, aromatische Säuren oder aliphatische oder aromatische Sulfonsäuren. Physiologisch unbedenkliche Salze dieser Säure sind daher z.B. das Sulfat, Pyrosulfat, Bisulfat, Sulfit, Bisulfit, Nitrat, Phosphat, Monohydrogenphosphat, Dihydrogenphosphat, Metaphosphat, Pyrophosphat, Chlorid, Bromid, Jodid, Fluorid, Acetat, Propionat, Decanoat, Caprylat, Acrylat, Formiat, Isobutyrat, Caproat, Heptanoat, Propiolat, Malonat, Succinat, Suberat, Sebacat, Fumarat, Maleat, Mandelat, Butin-1.4-dioat, Hexin-1.6-dioat, Benzoat, Chlorbenzoat, Methylbenzoat, Dinitrobenzoat, Hydroxybenzoat, Methoxybenzoat, Phthalat, Terephthalat, Benzolsulfonat, Toluolsulfonat, Chlorbenzolsulfonat, Xylolsulfonat, Phenylacetat, Phenylpropionat, Phenylbutyrat, Citrat, Lactat, $\beta$-Hydroxybutyrat, Glycollat, Malat, Tartrat, Methansulfonat, Propansulfonat, Naphthalin-1-sulfonat oder Naphthalin-2-sulfonat.

In Coll. Czech. Chem. Commun. 47, 1757 (1982) wird 3-(1,2-Diacetyl-6-methyl-8$\alpha$-ergolinyl)-1,1-diethylharnstoff und in EP-A-118848, EP-A-56358, Chem. Abstr. 105: 79.217$\mu$ (1986), EP-A-82808 und EP-A-160842 werden in 1- oder in 2-Stellung substituierte Ergoline beschrieben.

Im Vergleich zu den bekannten Ergolinen zeichnen sich die erfindungsgemäßen Verbindungen der Formel I durch eine zentral antidopaminerge Wirksamkeit und/oder $\alpha_2$-rezeptorblockierende Wirkung aus.

Die zentrale Dopaminrezeptorblockade wurde in einem Interaktionstest mit dem Dopaminrezeptoragonisten Apomorphin an Mäusen nach einmaliger i.p-Vorbehandlung dargestellt (Parameter: Aufhebung der durch Apomorphin 5 mg/kg i.p. verursachten Hypothermie). Männliche NMRI-Mäuse wurden mit verschiedenen Dosen der Substanz, die selbst nicht die Thermoregulation der Versuchstiere beeinflußen, bzw. mit Trägermedium vorbehandelt. 30 Minuten später erhielten alle Tiere Apomorphin 5 mg/kg i.p. 60 Minuten nach Zugabe von Substanz bzw. Trägermedium (= 30 Minuten nach Apomorphin) wurde die Rektaltemperatur mit Hilfe einer Thermosonde gemessen. Während die mit Trägermedium vorbehandelten Mäuse eine Hypothermie aufwiesen, war an mit Substanz vorbehandelten Tieren der körpertemperatursenkende Effekt des Apomorphin dosisabhängig aufgehoben.

Die zentrale $\alpha_2$-Rezeptorblockade wurde in einem Interaktionstest mit dem $\alpha_2$-Rezeptoragonisten Clonidin an Mäusen nach einmaliger i.p.-Vorbehandlung dargestellt (Parameter: Aufhebung der durch Clonidin 0,1 mg/kg i.p. verursachten Hypothermie). Männliche NMRI-Mäuse wurden mit verschiedenen Dosen der Substanz, die selbst nicht die Thermoregulation der Versuchstiere beeinflussen, bzw. mit Trägermedium vorbehandelt. 30 Minuten später erhielten alle Tiere Clonidin 0,1 mg/kg i.p., 60 Minuten nach Zugabe von Substanz bzw. Trägermedium (=30 Minuten nach Clonidin) wurde die Rektaltemperatur mit Hilfe einer Thermosonde gemessen. Während die mit Trägermedium vorbehandelten Mäuse eine Hypothermie aufwiesen, war an mit Substanz vorbehandelten Tieren der körpertemperatursenkende Effekt des Clondidin dosisabhängig aufgehoben.

Aufgrund dieser Befunde können die erfindungsgemäßen Verbindungen daher als Neuroleptika zur Behandlung von Psychosen des schizophrenen Formenkreises oder als Antidepressiva verwendet werden.

Die Herstellung der Verbindungen der allgemeinen Formel I erfolgt nach an sich bekannten Verfahren.

Das Verfahren zur Herstellung der Verbindungen der allgemeinen Formel I ist dadurch gekennzeichnet, daß man

a) eine Verbindung der allgemeinen Formel II

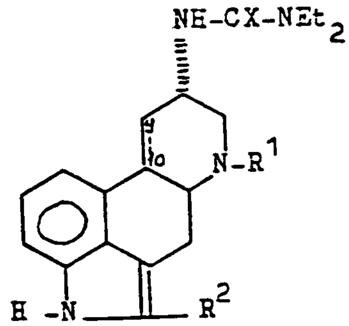

(II),

worin
X, $R^1$, $R^2$ und

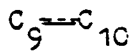

die obige Bedeutung haben, acyliert, oder. falls $R^2$ keine S-$R^5$- oder

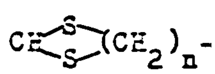

Gruppe mit $R^5$ und n in obiger Bedeutung ist, alkyliert,
b) eine Verbindung der allgemeinen Formel III

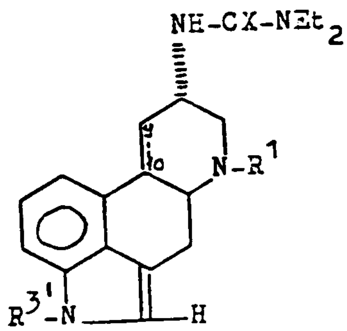

(III),

worin
X und $R^1$ die oben genannte Bedeutung haben und $R^{3'}$ Wasserstoff oder $C_{1-6}$- Alkyl bedeutet, thiomethyliert oder halogeniert und gegebenenfalls anschließend die so ererhaltenen Verbindungen der allgemeinen Formel I mit $R^2$ in der Bedeutung von Halogen

α) in das entsprechende 2-Lithiumergolinderivat überführt und anschließend mit einem Elektrophil zu Verbindungen der allgemeinen Formel I, worin $R^2$ $C_{1-6}$- Alkyl oder eine $SR^5$-Gruppe mit $R^5$ in der oben genannten Bedeutung darstellt, umsetzt,
oder
β) in Gegenwart eines Katalysators und einer Base mit einer ungesättigten gegebenenfalls wie oben beschrieben substituierten $C_{1-6}$- Alkylgruppe umsetzt zu Verbindungen der allgemeinen Formel I mit $R^2$ in der Bedeutung eines Alkenyl- oder Alkinylrestes und anschließend gegebenenfalls ganz oder partiell hydriert,

c) eine Verbindung der allgemeinen Formel III, worin X, $R^1$ und $R^{3'}$ die oben genannte Bedeutung haben, nach vorheriger Formylierung mit einem Dithiol umsetzt und gegebenenfalls anschließend die so erhaltenen Verbindungen mit $R^2$ in der Bedeutung von

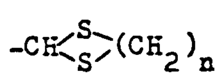

(n = 2 oder 3) reduktiv in die Methylgruppe überführt oder zur Formylgruppe hydrolysiert und diese

gegebenenfalls anschließend zu $-CH_2OH$ reduziert und die nach Verfahren b und c erhaltenen Verbindungen mit $R^{3'}$ = H acyliert
und die so erhaltenen Verbindungen gegebenenfalls in die Thioharnstoffe überführt und/oder die physiologisch verträglichen Säureadditionssalze bildet.

Die Substitution der Verbindungen der allgemeinen Formel II in 1-Stellung (Verfahren a) erfolgt zweckmäßigerweise nach der von V. Illi Synthesis 1979, 387 und Y. Kikugawa et al. Synthesis 1981 461 beschriebenen Methode, indem man im Zweiphasensystem das Ergolinderivat mit Alkylhalogeniden bzw. Carbonsäurechloriden oder -anhydriden umsetzt. Die Alkylierung kann auch in flüssigen Ammoniak nach F. Troxler et al. Helv. Chim. acta 40 1721 (1975) durchgeführt werden.

Sollen unsubstituierte Acetylenderivate in 1-Stellung alkyliert werden, so schützt man das Proton im allgemeinen durch eine der später aufgeführten üblichen Schutzgruppen.

Nach Verfahren b und c werden die gewünschten Substituenten in 2-Stellung eingeführt.

Die Halogenierung in 2-Stellung kann beispielsweise nach den in der Europäischen Patentanmeldung Nr. 0056358 beschriebenen Verfahren durchgeführt werden.

Die Methylthiolierung kann beispielsweise in einem inerten Lösungsmittel wie Dioxan oder Tetrahydrofuran mit einem Sulfonium-Salz, wie zum Beispiel das Dimethyl-methylthio-sulfoniumfluorborat, durchgeführt werden und ist nach 0,5 - 2 Stunden bei Raumtemperatur abgeschlossen.

Die Alkylthiolierung und Arylthiolierung kann jedoch auch über das 2-Lithiumergolinderivat und anschließender Umsetzung mit einem Schwefel-elektrophil vorgenommen werden.

Hierzu wird ein 2-Halogen-ergolin-derivat der Formel I mit einem Lithiumorganyl, bevorzugt Lithiumalkyl oder Lithiumphenyl, bei tiefen Temperaturen in einem aprotischen Lösungsmittel zum entsprechenden 2-Lithium-ergolin-derivat umgesetzt. Als Lithiumalkyl kommt insbesondere tert.-Butyl-lithium in Frage (EP-A-160-842).

Die anschließende elektrophile Substitution wird bei tiefen Temperaturen, vorzugsweise zwischen - 110 °C bis - 50 °C in dem gleichen aprotischen Lösungsmittel vorgenommen. Als aprotische Lösungsmittel sind Ether oder Kohlenwasserstoffe geeignet wie beispielsweise Tetrahydrofuran, Dioxan, Diethylether, Toluol, Hexan u.a.

Als Elektrophile seien bevorzugt genannt Thiosulfonsäure-S-ester wie zum Beispiel Methanthiosulfonsäure-S-methylester, Toluolthiosulfonsäure-S-ethylester, Toluolthiosulfonsäure-S-n-propylester oder Disulfide wie zum Beispiel Dibenzyldisulfid, Diphenyldisulfid, Tetraisopropylthiuramindisulfid oder niedere Alkylhalogenide wie beispielsweise Alkylbromide und -jodide oder halogenierte Silane wie beispielsweise niedere Alkylsilane wie Trimethylchlorsilan.
Zur Einführung der 2-Alkenyl- oder 2-Alkinylgruppe setzt man das 2-Halogen-ergolin-derivat mit einer monosubstituierten Acetylen- oder Vinylverbindung in Gegenwart eines Katalysators und einer Base um.

Die Umsetzung erfolgt bei Temperaturen von 0 °C bis 120 °C, bevorzugt 70 °C bis 100 °C, in einem aprotischen Lösungsmittel wie beispielsweise Dimethylformamid, N-Methylpyrrolidon, Tetrahydrofuran, Acetonitril oder Dioxan.

Als Katalysatoren werden bevorzugt Palladium-Verbindungen wie Palladiumsalze oder Palladium-Komplex-Verbindungen eingesetzt. Genannt seien zum Beispiel Palladium-II-acetat, trans-Dichlor-bis-(tri-o-tolyl-phosphin)-palladium(II) oder trans-Dichlor-bis-(triphenylphosphin)-palladium(II) und Palladium(O)-tetrakis-triphenylphosphin. Der Katalysator wird in einer Menge von 0,01 bis 0,1 Mol bezogen auf das eingesetzte 2-Ealogen-ergolin angewendet.

Zweckmäßigerweise wird die Reaktion unter Inertgasatmosphäre wie beispielsweise Stickstoff oder Argon, teilweise auch unter erhöhten Druck, durchgeführt.

Der Zusatz von Triarylphosphinen ist für die Reaktion förderlich.

Für die Ethinylierung sind katalytische Mengen von Kupfer-I-Salzen wie beispielsweise Kupfer-I-iodid oder Kupfer-I-bromid angebracht.

Als Basen sind sekundäre und tertiäre Amine geeignet wie zum Beispiel Dimethylamin, Diethylamin, Piperidin, Triethylamin und Tri-n-butylamin.

Sollen 2-Acetylen- oder 2-Vinyl-Ergolinderivate hergestellt werden, die im Acetylen- oder Vinylrest unsubstituiert sind, so schützt man zweckmäßigerweise ein Proton mit einer üblichen Schutzgruppe wie beispielsweise einer Trialkylsilylgruppe, die gegebenenfalls bei der Aufarbeitung des Reaktionsgemisches abgespalten wird.

Ist die Schutzgruppe der Tetrahydropyranylrest, so verwendet man zur Abspaltung eine Säure, wie zum Beispiel Pyridinium-p-toluolsulfonat oder verdünnte Schwefelsäure, in einem Alkohol bei 70 - 100 °C.

Mit katalytisch erregtem Wasserstoff können die 2-Vinylverbindungen ganz und die 2-Ethinylverbindungen ganz oder partiell hydriert werden.

Die völlige Hydrierung wird in Gegenwart eines Katalysators wie beispielsweise Raney-Nickel oder

Palladium auf verschiedenen Trägern wie Kohle bei Raumtemperatur gegebenenfalls unter erhöhtem Druck in einem inerten Lösungsmittel wie beispielsweise Alkoholen wie Methanol, Ethanol, Propanol, Ethern wie Dioxan, Diethylether, Säuren wie Eisessig u.a. durchgeführt.

Für die partielle Hydrierung zur Doppelbindung verwendet man als Katalysatoren beispielsweise mit Chinolin oder Pyridin aber auch mit Blei vergiftete Palladiumsalze oder Palladium auf diversen Trägern (Lindlar Katalysatoren). Als Lösungsmittel dienen die oben genannten Alkohole oder Kohlenwasserstoffe.

Eine Umwandlung der Ethinylverbindung in Vinylverbindungen kann aber auch durch Anlagerung von metallorganischen Verbindungen wie zum Beispiel Diisobutylaluminiumhydrid und anschließende Hydrolyse durchgeführt werden. Als Lösungsmittel kommen Kohlenwasserstoffe oder Aether in Frage.

Die entstehenden 2-Ethinyl- und 2-Hydroxy-propargyl-verbindungen können mit Alkylhalogeniden in Gegenwart von Lithiumdiiso-propylamid oder mit Phasentransferkatalysatoren zu Alkylethinylverbindungen bzw. Alkyl-propargylethern umgesetzt werden, wobei die 1-Stellung, falls sie unsubstituiert ist, mitalkyliert wird.

Die Herstellung von Verbindungen der allgemeinen Formel I mit $R^2$ in der Bedeutung von

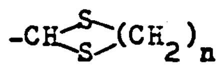

erfolgt beispielsweise durch Umsetzung von Verbindungen der allgemeinen Formel III mit Ameisensäureethylester und mit Lewis Säuren wie Ti-IV-chlorid bei Raumtemperatur in inerten Lösungsmitteln wie Chloroform, Methylenchlorid u.a.

Aus der Dithiolan-Verbindung kann man die 2-Methyl-verbindung durch Umsetzung mit Raney-Nickel bei Raumtemperatur in inerten Lösungsmitteln wie Alkoholen beispielsweise Methanol, Ethanol u.a. erhalten.

Die 2-Formylverbindung kann aus der Dithiolan-Verbindung erhalten werden, beispielsweise durch wässrige $SiO_2$-Behandlung und nachfolgender Umsetzung mit Sulfurylchlorid. Die Reaktion wird im allgemeinen bei Raumtemperatur in inerten Lösungsmitteln wie chlorierten Kohlenwasserstoffen, beispielsweise Chloroform, Methylenchlorid, Ethylenchlorid u.a. durchgeführt.

Der Aldehyd kann zum entsprechenden Alkohol reduziert werden, beispielsweise mit $LiAlH_4$ in inerten Lösungsmitteln wie Ethern z.B. Tetrahydrofuran, Diethylether u.a. oder mit $NaBH_4$ in Lösungsmitteln wie Alkoholen, zum Beispiel Methanol, Ethanol u.a.

Die Überführung der 8α-Harnstoffderivate in die entsprechenden Thione erfolgt durch Umsetzung mit Phosphoroxychlorid und nachfolgender Reaktion mit Kaliumxanthogenat. Die Reaktion wird bei tiefen Temperaturen mit zwischenzeitlicher Temperaturerhöhung in inerten Lösungsmitteln wie Ethern vorgenommen.

Die Thioharnstoffderivate können auch durch Spaltung des Harnstoffes und anschließende Umsetzung des Amins mit Thiostaabreagenz und einem weiteren Amin hergestellt werden.

Alle Reaktionen werden im allgemeinen unter Schutzgasatmosphäre wie Argon oder Stickstoff durchgeführt.

Zur Bildung von Salzen werden die Verbindungen der Formel I in wenig Methanol oder Methylenchlorid gelöst und mit einer konzentrierten Lösung der gewünschten Säure in Methanol bei Raumtemperatur versetzt.

Zur Verwendung der erfindungsgemäßen Verbindungen als Arzneimittel werden diese in die Form eines pharmazeutischen Präparats gebracht, das neben dem Wirkstoff ein für die enterale oder parentale Applikation geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüberhinaus Hilfsstoffe wie Konservierungs, Stabilisierungs-, Netzmittel oder Emulgatoren, Salze zur Veränderung des osmotischen Drucks oder Puffer.

Die Herstellung der Ausgangsverbindungen ist bekannt oder erfolgt nach bekannten Methoden.

Die nachfolgenden Beispiele sollen das erfindungsgemäße Verfahren erläutern.

Beispiel 1

3-(2-Brom-1,6-dimethyl-8α-ergolinyl)-1,1-diethyl-harnstoff

Man löst 4,18 g 3-(2-Brom-6-methyl-8α-ergolinyl)-1,1-diethylharnstoff (10 mMol) in 50 ml THF, gibt 40 mg Tetrabutylammoniumhydrogensulfat, 1 g pulverisiertes Kaliumhydroxid und 2 ml Methyljodid zu und

rührt 4 Stunden bei Raumtemperatur. Dann gießt man die organische Phase ab, schüttelt sie mit gesättigter Bicarbonatlösung aus und dampft nach Trocknen mit Natriumsulfat ein. Der Rückstand wird aus Essigester kristallisiert, Ausbeute 2,89 g (67 % d. Th).
$[\alpha]_D$ = + 10 ° (0,5 % in Chloroform)
In analoger Weise werden die folgenden 1-Alkylierungen durchgeführt:
Aus 3-(2-Brom-6-n-propyl-8α-ergolinyl)-1,1-diethyl-harnstoff der 3-(2-Brom-1-methyl-6-n-propyl-8α-ergolinyl)-1,1-diethylharnstoff, kristallisiert als weinsaures Salz, Aubeute 63 %.
$[\alpha]_D$ = + 2 ° (0.5 % in Methanol)
1,1-Diethyl-3-(1-propyl-2-jod-6-methyl-8α-ergolinyl)-harnstoff, Ausbeute 77 %.
$[\alpha]_D$ = + 33.6 °,C = 0,205; Pyridin
1,1-Diethyl-3-(1-methyl-2-ethinyl-6-methyl-8α-ergolinyl)-harnstoff über die Trimethylsilylethinylverbindung und anschliessende Abspaltung der Trimethylsilylgruppe mit ethanolisch wässriger Kaliumcarbonatlösung, Schmelzpunkt 114 - 117 °C.
1,1-Diethyl-3-(1-propyl-2-ethyl-6-methyl-8α-ergolinyl)-harnstoff
1,1-Diethyl-3-(1-methyl-2-phenethyl-6-methyl-8α-ergolinyl)-harnstoff
1,1-Diethyl-3-(1-propyl-2-phenethyl-6-methyl-8α-ergolinyl)-harnstoff
1,1-Diethyl-3-(1-methyl-2-phenylethinyl-6-methyl-8α-ergolinyl)-harnstoff
1,1-Diethyl-3-[1-methyl-2-(3-methoxypropinyl)-6-methyl-8α-ergolinyl]-harnstoff

## Beispiel 2

### 1,1-Diethyl-3-(1,6-dimethyl-2-methylthio-8α-ergolinyl)-harnstoff

Man löst 354 mg 1,1-Diethyl-3-(1,6-dimethyl-8α-ergolinyl)-harnstoff (1 mMol) in 20 ml THF und fügt bei Raumtemperatur unter Stickstoff 392 mg Dimethyl-methylthio-sulfoniumtetrafluorborat (2 mMol) und 0,24 ml 50 %ige Tetrafluorborsäure zu. Nach 30 Minuten Rühren bei Raumtemperatur verteilt man zwischen Methylenchlorid und Bicarbonatlösung, vereinigt die organischen Phasen, trocknet sie mit Natriumsulfat und dampft ein. Das Rohprodukt wird mit Methylenchlorid an Kieselgel chromatographiert, die Ausbeute beträgt nach Kristallisation 276 mg (69 % d. Th.).
$[\alpha]_D$ = +11 ° (0,5 % in Chloroform)
Aus 3-(9,10-Didehydro-1,6-dimethyl-8α-ergolinyl)-1,1-diethylharnstoff erhält man nach der obigen Vorschrift in Anwesenheit von Tetrafluorborsäure den 3-(9,10-Didehydro-1,6-dimethyl-2-methylthio-8α-ergolinyl)-1,1-diethyl-harnstoff in 55 % Ausbeute.

## Beispiel 3

### 3-(2-Brom-9,10-didehydro-1,6-dimethyl-8α-ergolinyl)-1,1-diethyl-harnstoff

Man löst 352 mg 3-(9,10-Didehydro-1,6-dimethyl-8α-ergolinyl)-1,1-diethyl-harnstoff (1 mMol) in 20 ml wasserfreiem Dioxan, versetzt mit 484 mg Pyrrolidonhydroperbromid (1,5 mMol) und rührt 30 Minuten bei Raumtemperatur. Anschließend gießt man das Reaktionsgemisch in eine gesättigte Bicarbonatlösung, extrahiert mit Methylenchlorid und trocknet die organische Phase mit Natriumsulfat. Nach Abdampfen des Lösungsmittels wird der Rückstand an Kieselgel chromatographiert und man isoliert nach Kristallisation 345 mg der gewünschten Verbindung (80 % d.Th.).
$[\alpha]_D$ = + 299 ° (0,5 % in Chloroform)

## Beispiel 4

### 1,1-Diethyl-3-(1,6-dimethyl-2-phenylthio-8α-ergolinyl)-harnstoff

Zu 4 ml wasserfreiem frisch destillierten Toluol gibt man unter Argon 0,3 ml (1,5 mMol) destilliertes Hexamethyldisilazan und kühlt die Mischung auf 0 °C ab. Dann werden 0,85ml (1,4 mMol) 15 %iges Butyllithium in Hexan zugetropft und 15 Minuten bei 0 °C nachgerührt. Dazu gibt man die Lösung von 432 mg 3-(2-Brom-1,6-dimethyl-8α-ergolinyl)-1,1-diethylharnstoff (1 mMol) in 50 ml wasserfreiem, frisch destillierten Toluol und rührt weitere 15 Minuten bei 0 °C nach. Nach der Zugabe von 1 ml destilliertem Tetramethylethylendiamin wird der Ansatz auf - 90 °C abgekühlt. Bei dieser Temperatur gibt man 5 ml (7 mMol) tert.-Butyllithium (14 % in Hexan) zu und rührt 2 Minuten nach, bevor man 1 g Diphenyldisulfid (5 mMol) zugibt. Nach 2 Stunden Rühren bei - 70 °C wird zur Aufarbeitung Wasser zugegeben. Die Extraktion

erfolgt mit Essigester. Die organischen Phasen werden mit gesättigter Natriumhydrogencarbonatlösung und Wasser gewaschen. Die vereinigten Essigesterphasen werden über Natriumsulfat getrocknet und eingeengt. Nach Kristallisation aus Essigester und tert.-Butylmethylether erhält man 180 mg der gewünschten Verbindung (39 % d. Th.).

In völlig analoger Weise werden aus dem gleichen Ausgangsmaterial die folgenden Harnstoffe dargestellt:

Mit Dibenzyldisulfid der 3-(2-Benzylthio-1,6-dimethyl-8α-ergolinyl)-1,1-diethyl-harnstoff, Ausbeute 47 %.

Mit p-Toluolthiosulfonsäure-S-n-propylester der 1,1-Diethyl-3-(1,6-dimethyl-2-n-proylthio-8α-ergolinyl)-harnstoff, Ausbeute 65 %.

Setzt man 3-(2-Brom-9,10-didehydro-1,6-dimethyl-8α-ergolinyl)-1,1-diethyl-harnstoff mit Diphenyldisulfid um, erhält man 3-(9,10-Didehydro-1,6-dimethyl-2-phenylthio-8α-ergolinyl)-1,1-diethyl-harnstoff in 47 % Ausbeute.

Beispiel 5

Zu einer Lösung von 6,9 g 1,1-Diethyl-3-(1,6-dimethyl-8α-ergolinyl)-harnstoff in 200 ml Chloroform gibt man bei Raumtemperatur unter Argon nacheinander 7 ml Ameisensäureethylester und 3,6 ml (44 mMol) Ethandithiol. Danach tropft man langsam 8,8 ml (80 mMol) Titan-IV-chlorid in 100 ml Chloroform gelöst dazu. Es werden 20 Stunden bei Raumtemperatur nachgerührt. Obwohl das Ausgangsmaterial zu diesem Zeitpunkt noch nicht vollständig umgesetzt ist, wird das Reaktionsgemisch aufgearbeitet, um eine weitere Entstehung der di-substituierten Verbindung zu vermeiden. Hierzu wird das Reaktionsgemisch im Eisbad gekühlt und nacheinander tropfenweise mit 50 ml Methanol und 40 ml Wasser versetzt. Dann wird die Mischung mit 25 %iger Ammoniaklösung alkalisiert und mit Methylenchlorid extrahiert. Die organischen Phasen wäscht man mit Wasser und trocknet sie über Magnesiumsulfat. Der eingedampfte Rückstand wird in der Siedehitze aus Methanol/Essigester kristallisiert. Man erhält 3,0 g 1,1-Diethyl-3-[2-(1,3-dithiolan2-yl-)-1,6-dimethyl-8α-ergolinyl]-harnstoff (37 % Ausbeute).

Beispiel 6

7,5 ml einer Raney-Nickel-Suspension werden viermal mit je 30 ml Methanol gewaschen. Dann gibt man 15 ml Methanol und anschließend eine Lösung aus 690 mg (1,5 mMol) 1,1-Diethyl-3-[2-(1,3-dithiolan-2-yl)-1,6-dimethyl-8α-ergolinyl]-harnstoff in 15 ml Methanol dazu. Man rührt 3 Stunden bei Raumtemperatur nach und gibt nochmals 7,5 ml einer Raney-Nickel-Suspension zu, die man vorher wie oben viermal mit je 30 ml Methanol gewaschen hat. Nach weiteren 2 Stunden Rühren bei Raumtemperatur wird der Katalysator über eine Kieselgelsäule abfiltriert und mit Methanol gründlich gewaschen. Das Filtrat wird eingedampft und der Rückstand aus Methanol/Essigester kristallisiert. Man erhält 150 mg 1,1-Diethyl-3-(1,2,6-trimethyl-8α-ergolinyl)-harnstoff (27 % Ausbeute).

Beispiel 7

2,12 g (5 mMol) 1,1-Diethyl-3-[2-(1,3-dithiolan-2-yl)-6-methyl-8α-ergolinyl]-harnstoff werden in 40 ml Chloroform unter Argon gelöst. Dann gibt man 3,5 g Kieselgel und unter kräftigem Rühren tropfenweise 3.5 ml Wasser dazu. Während eines Zeitraumes von 30 Minuten tropft man eine Lösung aus 1,18 ml Sulfurylchlorid in 30 ml Chloroform zu. Nach 3 Stunden Rühren bei Raumtemperatur werden 7,5 g Kaliumcarbonat zugegeben und 20 Minuten kräftig gerührt. Der Niederschlag wird abfiltriert und mit Chloroform nachgewaschen. Den Niederschlag befeuchtet man mit Ethanol und gibt ihn in 300 ml gesättigte Kochsalzlösung, welche man dann mit Chloroform mehrmals extrahiert. Die Chloroformextrakte und das Chloroformfiltrat werden gemeisam über Magnesiumsulfat getrocknet und eingedampft. Die anschließende Kristallisation erfolgt aus Essigester. Es werden 1,16 g 1,1-Diethyl-3-(6-methyl-2-formyl-8α-ergolinyl)-harnstoff (61 % Ausbeute) isoliert.

Beispiel 8

320 mg (8 mMol) Lithiumaluminiumhydrid suspendiert man unter Argon in 20 ml absolutem frisch destillierten Tetrahydrofuran. Bei Raumtemperatur wird eine Lösung aus 1,53 g (4 mMol) 1,1-Diethyl-3-(1,6-dimethyl-2-formyl-8α-ergolinyl)-harnstoff in 40 ml frisch destilliertem, absolutem Tetrahydrofuran zugetropft. Anschließend rührt man 1 1/4 Stunden bei Raumtemperatur. Der Ansatz wird dann im Eisbad abgekühlt und mit 20 ml 1n Salzsäure versetzt. Man gibt 20 ml 2n-Weinsäure dazu und überschichtet den Ansatz mit

Essigester. Zur Neutralisation werden 80 ml 1n Ammoniaklösung zugetropft. Die Essigesterphase wird abgetrennt und die wässrige Phase mit Essigester nachextrahiert. Die vereinigten Essigesterphasen trocknet man über Natriumsulfat. Das eingeengte Rohprodukt wird an Kieselgel mit Methylenchlorid/Methanol 95:5 unter Druck chromatographiert. Das Rohprodukt (1,0 g) wird aus Essigester kristallisiert, wobei man 0,72 g 1,1-Diethyl-3-(1,6-dimethyl-2-hydroxymethyl-8α-ergolinyl)-harnstoff (47 % Ausbeute) erhält.

Beispiel 9

1,1-Diethyl-3-(2-jod-1-acetyl-6-methyl-8α-ergolinyl)-harnstoff

1,05 g 1,1-Diethyl-3-(2-jod-6-methyl-8α-ergolinyl)-harnstoff werden in 90 ml Methylenchlorid mit 115 mg Tetrabutylammoniumhydrogensulfat, 0,18 ml Acetylchlorid und 139 mg gepulverter Kaliumhydroxydplätzchen 3 Stunden bei Raumtemperatur gerührt. Nach Absaugen über Kieselgur wird nochmals mit den gleichen Mengen Acetylchlorid, gepulvertem Kaliumhydroxid und Tetrabutylammoniumhydrogensulfatversetzt und 1 Stunde bei Raumtemperatur gerührt. Nach Versetzen mit Eiswasser wird mit Natriumhydrogenkarbonat neutralisiert und ausgeschüttelt. Die organische Phase wird getrocknet, filtriert und eingeengt. Der Rückstand wird über Kieselgel mit Methylenchlorid : Ethanol = 10 : 1 als Elutionsmittel chromatographiert. Man erhält 500 mg 1,1-Diethyl-3-(2-jod-1-acetyl-6-methyl-8α-ergolinyl)-harnstoff als Öl.
$[\alpha]_D$ = + 40 ° (C = 0,2 Pyridin)
In analoger Weise werden hergestellt:
1,1-Diethyl-3-[1-acetyl-2-{(3-tetrahydropyran-2-yl-oxy)-1-propinyl}-6-methyl-8α-ergolinyl]-harnstoff

Beispiel 10

1,1-Diethyl-3-(2-vinyl-1-propyl-6-methyl-8α-ergolinyl)-harnstoff

1,15 g 1,1-Diethyl-3-(2-jod-1-propyl-6-methyl-8α-ergolinyl)-harnstoff werden in 10 ml Dimethylformamid mit 10 ml Triäthylamin, 53,5 mg Triorthotolylphosphin, 39,4 mg Palladium(II)-chlorid und 1,34 ml Vinyltrimethysilan 3 Stunden bei 115 °C Badtemperatur in einem Kleinautoklaven unter Argon erwärmt. Nach Eindampfen wird in Methylenchlorid/Natriumbicarbonatlösung verteilt. Die organische Phase wird eingedampft und über Kieselgel mit Essigester : Ethanol = 3 : 1 als Elutionsmittel chromatographiert. Man erhält 218 mg 1,1-Diethyl-3-(2-trimethylsilylvinyl -1-propyl-6-methyl-8α-ergolinyl)-harnstoff und 230 mg 1,1-Diethyl-3-(2-Vinyl-1-propyl-6-methyl-8α-ergolinyl)-harnstoff als Öle.

Beispiel 11

1,1-Diethyl-3-(2-ethyl-1-propyl-6-methyl-8α-ergolinyl)-harnstoff

150 mg 1,1-Diethyl-3-(2-vinyl-1-propyl-6-methyl-8α-ergolinyl)-harnstoff werden in 20 ml Ethanol mit 0,1 g Raney-Nickel (B 115 Z) bei Raumtemperatur und Normalwasserstoffdruck hydriert. Nach Abfiltrieren des Katalysators über Kieselgur, Eindampfen des Filtrates und Chromatographie über Kieselgel mit Toluol : Ethanol : Wasser = 80 : 20 : 1 als Elutionsmittel erhält man 65 mg 1,1-Diethyl-3-(2-ethyl-1-propyl-6-methyl-8α-ergolinyl)-harnstoff als Öl.

Beispiel 12

3-(6-Methyl-1-propyl-2-ethyl-8α-ergolinyl)-1,1-diethylthioharnstoff

200 mg 1,1-Diethyl-3-(2-ethyl-6-methyl-1-propyl-8α-ergolinyl)-harnstoff werden in 10 ml Methylenchlorid bei - 12 °C mit 0,13 ml Phosphoroxychlorid versetzt. In 1 Stunde läßt man den Ansatz auf Raumtemperatur erwärmen und rührt bei dieser Temperatur 16 Stunden. Nach Abziehen wird mit Aether ausgerührt, abgesaugt und im Vakuum über KOH-Plätzchen getrocknet. Das getrocknete Material wird dann mit einer Lösung von 245 mg Kaliumxanthogenat in 15 ml Acetonitril bei - 10 °C versetzt und 2 Stunden bei Raumtemperatur nachgerührt. Nach Eindampfen wird in Methylenchlorid/Natriumhydrogenkarbonatlösung verteilt, die organische Phase eingeengt und der Rückstand über Kieselgel mit Methylenchlorid : Ethanol = 10 : 1 als Elutionsmittel chromatographiert. Nach Umkristallisation aus wenig Ethanol/Hexan erhält man 100 mg 3-(6-Methyl-1-propyl-2-ethyl-8α-ergolinyl)-1,1-diethylthioharnstoff vom Schmelzpunkt 173 - 175 °C.

$[\alpha]_D = +48°$ (C = 0,2 Pyridin)

Beispiel 13

1,1-Diethyl-3-(2-trimethylsilylethinyl-1-acetyl-6-methyl-8α-ergolinyl)-harnstoff

260 mg 1,1-Diethyl-3-(2-jod-1-acetyl-6-methyl-8α-ergolinyl)-harnstoff werden in 3 ml Dimethylformamid mit 6 ml Triäthylamin, 0,5 ml Trimethylsilylacetylen, 6 mg Kupfer(I)jodid und 15 mg Bistriorthotolylphosphinpalladium(II)dichlorid unter Argon 1,5 Stunden auf 60 °C erwärmt. Nach Eindampfen wird in Essigester/Bicarbonatlösung verteilt. Die organische Phase wird nach Eindampfen über Kieselgel mit Methylenchlorid : Methanol = 9 : 1 chromatographiert. Man erhält 100 mg 1,1-Diethyl-3-(2-trimethylsilylethinyl-1-acetyl-6-methyl-8α-ergolinyl)-harnstoff als Öl.

Beispiel 14

1,1-Diethyl-3-(2-ethinyl-1-propyl-6-methyl-8α-ergolinyl)-harnstoff

109 mg 1,1-Diethyl-3-(2-ethinyl-6-methyl-8α-ergolinyl)-harnstoff werden in 5 ml absolutem Tetrahydrofuran bei - 30 °C unter Argon zu einer aus 0,15 ml Diisopropylamin und 1,8 ml 0,6 molaren n-Butyllithiumlösung (Hexan) in 3 ml absolutem Tetrahydrofuran bei 0 °C in 15 Minuten bereiten Lösung von Lithiumdiisopropylamid getropft. Nach 30 Minuten Rühren bei - 30 °C werden 0,07 ml Propyljodid zugegeben. Es wird 1 Stunde bei - 20 °C und über Nacht bei Raumtemperatur gerührt. Nach Eindampfen wird nach Zugabe von Natriumbicarbonatlösung und wenig Ethanol mit Essigester ausgeschüttelt. Die organische Phase wird eingeengt und über Kieselgel mit Toluol :Eisessig : Wasser = 10 : 10 : 1 chromatographiert. Man erhält 30 mg 1,1-Diethyl-3-(2-ethinyl-1-propyl-6-methyl-8α-ergolinyl)-harnstoff als Öl.

Nach dem gleichen Verfahren erhält man aus dem gleichen Ausgangsmaterial wie oben 1,1-Diethyl-3-(2-propinyl-1,6-dimethyl-8α-ergolinyl)-harnstoff als Öl.

**Patentansprüche**

**1.** 1,2-disubstituierte Ergolinderivate der allgemeinen Formel I

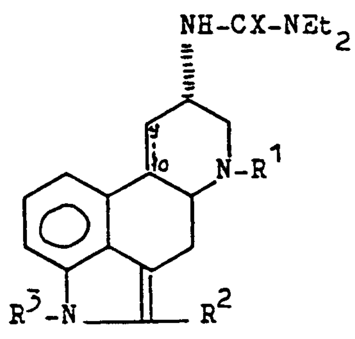

(I),

worin

X ein Sauerstoff- oder Schwefelatom bedeutet,

$R^1$ eine $C_{1-6}$-Alkylgruppe ist

$R^2$ Halogen, eine gesättigte oder ungesättigte $C_{1-6}$-Alkylgruppe, die gegebenenfalls substituiert sein kann mit $OR^4$ oder einem gegebenenfalls mit $C_{1-2}$-Alkyl, $C_{1-2}$-Alkoxy oder Halogen substituierten Phenyl-, Pyridin-, Thiophen-, Furan-, Pyrimidin-, Imidazol- oder Pyrazol-Rest, wobei $R^4$ Wasserstoff, $C_{1-6}$-Alkyl, Tetrahydropyranyl oder $C_{5-6}$-Cycloalkyl bedeutet,

eine $S-R^5$-Gruppe, worin $R^5$ eine $C_{1-6}$-Alkylgruppe, die gegebenenfalls mit Phenyl substituiert sein kann oder einen gegebenenfalls mit $C_{1-2}$-Alkyl, $C_{1-2}$-Alkoxy oder Halogen substituierten Phenyl-, Pyridin-, Thiophen-, Furan-, Pyrimidin-, Imidazol- oder Pyrazol-Rest darstellt,

eine

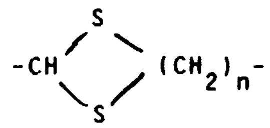

Gruppe, worin n 2 oder 3 ist oder

eine CHO-Gruppe bedeutet,

$R^3$ $C_{1-6}$-Alkyl oder $C_{2-4}$-Acyl ist und

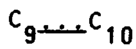

eine CC-Einfach- oder eine CC-Doppelbindung bedeutet und das Wasserstoffatom in 10-Stellung α-ständig ist, wenn

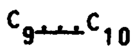

eine CC-Einfachbindung darstellt, sowie deren Säureadditionssalze.

**2.** 3-(2-Brom-1,6-dimethyl-8α-ergolinyl)-1,1-diethyl-harnstoff

3-(2-Brom-1-methyl-6-n-propyl-8α-ergolinyl)-1,1-diethyl-harnstoff

1,1-Diethyl-3-(1-propyl-2-jod-6-methyl-8α-ergolinyl)-harnstoff

1,1-Diethyl-3-(1-methyl-2-ethinyl-6-methyl-8α-ergolinyl)-harnstoff

1,1-Diethyl-3-(1-propyl-2-ethyl-6-methyl-8α-ergolinyl)-harnstoff

1,1-Diethyl-3-(1-methyl-2-phenethyl-6-methyl-8α-ergolinyl)-harnstoff

1,1-Diethyl-3-(1-methyl-2-phenylethinyl-6-methyl-8α-ergolinyl)-harnstoff

1,1-Diethyl-3-[1-methyl-2-(3-methoxypropinyl)-6-methyl-8α-ergolinyl]-harnstoff

1,1-Diethyl-3-(1,6-dimethyl-2-methylthio-8α-ergolinyl)-harnstoff

3-(9,10-Didehydro-1,6-dimethyl-2-methylthio-8α-ergolinyl)-1,1-diethyl-harnstoff

3-(2-Brom-9,10-didehydro-1,6-dimethyl-8α-ergolinyl)-1,1diethyl-harnstoff

1,1-Diethyl-3-(1,6-dimethyl-2-phenylthio-8α-ergolinyl)-harnstoff

3-(2-Benzylthio-1,6-dimethyl-8α-ergolinyl)-1,1-diethyl-harnstoff

1,1-Diethyl-3-(1,6-dimethyl-2-n-propylthio-8α-ergolinyl)-harnstoff

3-(9,10-Didehydro-1,6-dimethyl-2-phenylthio-8α-ergolinyl)-1,1-diethyl-harnstoff

1,1-Diethyl-3-[2-(1,3-dithiolan-2-yl)-1,6-dimethyl-8α-ergolinyl]-harnstoff

1,1-Diethyl-3-(1,2,6-trimethyl-8α-ergolinyl)-harnstoff

1,1-Diethyl-3-(6-methyl-2-formyl-8α-ergolinyl)-harnstoff

1,1-Diethyl-3-(1,6-dimethyl-2-hydroxymethyl-8α-ergolinyl)-harnstoff

1,1-Diethyl-3-(2-jod-1-acetyl-6-methyl-8α-ergolinyl)-harnstoff

1,1-Diethyl-3-[1-acetyl-2-{(3-tetrahydropyran-2-yl-oxy)-1-propinyl}-6-methyl-8α-ergolinyl]-harnstoff

1,1-Diethyl-3-(2-vinyl-1-propyl-6-methyl-8α-ergolinyl)-harnstoff

1,1-Diethyl-3-(2-ethyl-1-propyl-6-methyl-8α-ergolinyl)-harnstoff

3-(6-Methyl-1-propyl-2-ethyl-8α-ergolinyl)-1,1-diethyl-thioharnstoff

1,1-Diethyl-3-(2-ethinyl-1-propyl-6-methyl-8α-ergolinyl)-harnstoff

1,1-Diethyl-3-(2-propinyl-1,6-dimethyl-8α-ergolinyl)-harnstoff nach Anspruch 1.

**3.** Verfahren zur Herstellung der Verbindungen der allgemeinen Formel I, dadurch gekennzeichnet, daß man
a) eine Verbindung der allgemeinen Formel II

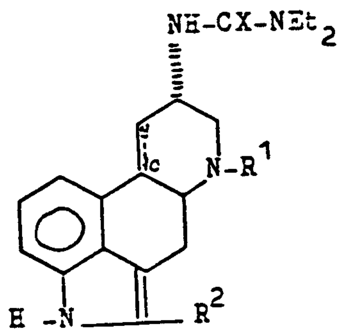

(II),

worin
X, $R^1$, $R^2$ und

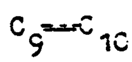

die obige Bedeutung haben, acyliert, oder. falls $R^2$ keine S-$R^5$- oder

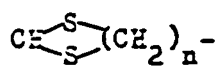

Gruppe mit $R^5$ und n in obiger Bedeutung ist, alkyliert,
b) eine Verbindung der allgemeinen Formel III

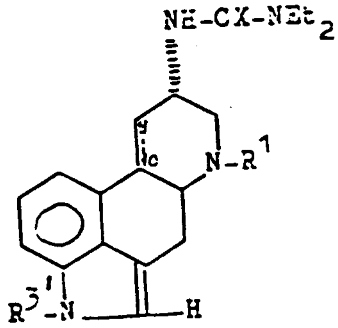

(III),

worin

X und $R^1$ die oben genannte Bedeutung haben und $R^{3'}$ Wasserstoff oder $C_{1-6}$- Alkyl bedeutet, thiomethyliert oder halogeniert und gegebenenfalls anschließend die so ererhaltenen Verbindungen der allgemeinen Formel I mit $R^2$ in der Bedeutung von Halogen

α) in das entsprechende 2-Lithiumergolinderivat überführt und anschließend mit einem Elektrophil zu Verbindungen der allgemeinen Formel I, worin $R^2$ $C_{1-6}$- Alkyl oder eine $SR^5$-Gruppe mit $R^5$ in der oben genannten Bedeutung darstellt, umsetzt,

oder

β) in Gegenwart eines Katalysators und einer Base mit einer ungesättigten gegebenenfalls wie oben beschrieben substituierten $C_{1-6}$- Alkylgruppe umsetzt zu Verbindungen der allgemeinen Formel I mit $R^2$ in der Bedeutung eines Alkenyl- oder Alkinylrestes und anschließend gegebenenfalls ganz oder partiell hydriert,

c) eine Verbindung der allgemeinen Formel III, worin X, $R^1$ und $R^{3'}$ die oben genannte Bedeutung haben, nach vorheriger Formylierung mit einem Dithiol umsetzt und gegebenenfalls anschließend die so erhaltenen Verbindungen mit $R^2$ in der Bedeutung von

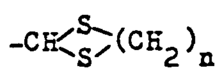

(n = 2 oder 3) reduktiv in die Methylgruppe überführt oder zur Formylgruppe hydrolysiert und diese gegebenenfalls anschließend zu $-CH_2OH$ reduziert und die nach Verfahren b und c erhaltenen Verbindungen mit $R^{3'}$ = H acyliert

und die so erhaltenen Verbindungen gegebenenfalls in die Thioharnstoffe überführt und/oder die physiologisch verträglichen Säureadditionssalze bildet.

**4.** Verwendung der Verbindungen nach Anspruch 1 und 2 zur Herstellung von Arzneimittel.

## Claims

**1.** 1,2-disubstituted ergoline derivatives of the general formula I

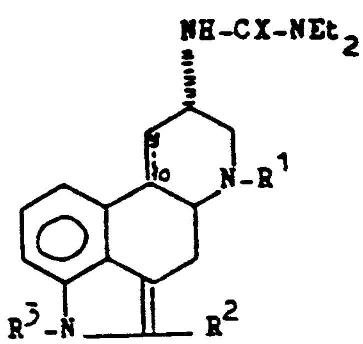

(I)

wherein

X represents an oxygen or sulphur atom,

$R^1$ is a $C_{1-6}$ alkyl group,

$R^2$ represents halogen, a saturated or unsaturated $C_{1-6}$alkyl group that may optionally be substituted by $OR^4$ or by a phenyl, pyridinyl, thiophenyl, furanyl, pyrimidinyl, imidazolyl or pyrazolyl radical each of which is optionally substituted by $C_{1-2}$alkyl, $C_{1-2}$alkoxy or by halogen, wherein $R^4$ represents hydrogen, $C_{1-6}$alkyl, tetrahydropyranyl or $C_{5-6}$cycloalkyl,

an S-$R^5$ group wherein $R^5$ represents a $C_{1-6}$alkyl group which may optionally be substituted by phenyl, or a phenyl, pyridinyl, thiophenyl, furanyl, pyrimidinyl, imidazolyl or pyrazolyl radical each of which is optionally substituted by $C_{1-2}$alkyl, $C_{1-2}$alkoxy or by halogen,

a

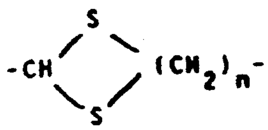

group, wherein n is 2 or 3, or

a CHO group;

$R^3$ is $C_{1-6}$alkyl or $C_{2-4}$acyl and

$C_9 \ldots C_{10}$

represents a CC-single bond or a CC-double bond and the hydrogen atom in the 10-position is in the α-configuration when

$C_9 \ldots C_{10}$

represents a CC-single bond, and the addition salts thereof.

**2.** 3-(2-bromo-1,6-dimethyl-8α-ergolinyl)-1,1-diethylurea
3-(2-bromo-1-methyl-6-n-propyl-8α-ergolinyl)-1,1-diethylurea
1,1-diethyl-3-(1-propyl-2-iodo-6-methyl-8α-ergolinyl)urea
1,1-diethyl-3-(1-methyl-2-ethynyl-6-methyl-8α-ergolinyl)urea
1,1-diethyl-3-(1-propyl-2-ethyl-6-methyl-8α-ergolinyl)urea
1,1-diethyl-3-(1-methyl-2-phenethyl-6-methyl-8α-ergolinyl)urea
1,1-diethyl-3-(1-methyl-2-phenylethynyl-6-methyl-8α-ergolinyl)urea
1,1-diethy1-3-[1-methyl-2-(3-methoxypropynyl)-6-methyl-8α-ergolinyl]urea
1,1-diethyl-3-(1,6-dimethyl-2-methylthio-8α-ergolinyl)urea
3-(9,10-didehydro-1,6-dimethyl-2-methylthio-8α-ergolinyl)-1,1-diethylurea
3-(2-bromo-9,10-didehydro-1,6-dimethyl-8α-ergolinyl)-1,1-diethylurea
1,1-diethyl-3-(1,6-dimethyl-2-phenylthio-8α-ergolinyl)urea
3-(2-benzylthio-1,6-dimethyl-8α-ergolinyl)-1,1-diethylurea
1,1-diethyl-3-(1,6-dimethyl-2-n-propylthio-8α-ergolinyl)urea
3-(9,10-didehydro-1,6-dimethyl-2-phenylthio-8α-ergolinyl)-1,1-diethylurea
1,1-diethyl-3-[2-(1,3-dithiolan-2-yl)-1,6-dimethyl-8α-ergolinyl]urea
1,1-diethyl-3-(1,2,6-trimethyl-8α-ergolinyl)urea
1,1-diethyl-3-(6-methyl-2-formyl-8α-ergolinyl)-urea
1,1-diethyl-3-(1,6-dimethyl-2-hydroxymethyl-8α-ergolinyl)urea
1,1-diethyl-3-(2-iodo-1-acetyl-6-methyl-8α-ergolinyl)urea
1,1-diethyl-3-[1-acetyl-2-((3-tetrahydropyran-2-yloxy)-1-propynyl)-6-methyl-8α-ergolinyl]urea
1,1-diethyl-3-(2-vinyl-1-propyl-6-methyl-8α-ergolinyl)urea
1,1-diethyl-3-(2-ethyl-1-propyl-6-methyl-8α-ergolinyl)urea
3-(6-methyl-1-propyl-2-ethyl-8α-ergolinyl)-1,1-diethylthiourea
1,1-diethyl-3-(2-ethynyl-1-propyl-6-methyl-8α-ergolinyl)urea

1,1-diethyl-3-(2-propynyl-1,6-dimethyl-8α-ergolinyl)urea according to claim 1.

**3.** Process for the preparation of compounds of the general formula I, characterized in that

a) a compound of the general formula II

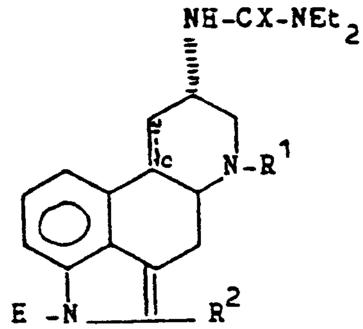

(II)

wherein X, $R^1$, $R^2$ and

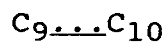

have the meanings given above, is acylated or, if $R^2$ is not an S-$R^5$- or

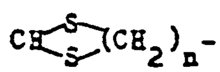

group wherein $R^5$ and n have the meanings given above, is alkylated,

b) a compound of the general formula III

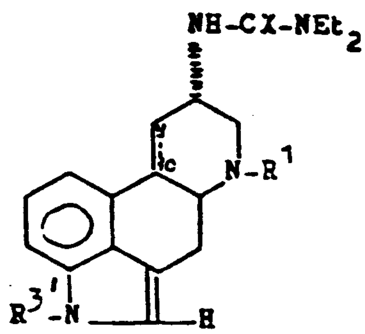

(III)

wherein

X and $R^1$ have the meanings given above and $R^{3'}$ represents hydrogen or $C_{1-6}$alkyl, is thiomethylated or halogenated, and optionally then the resulting compounds of the general formula I in which $R^2$ represents halogen

α) are converted into the corresponding 2-lithium ergoline derivative and then reacted with an electrophile to yield compounds of the general formula I wherein $R^2$ represents $C_{1-6}$alkyl or an $SR^5$ group wherein $R^5$ has the meaning given above, or

β) are reacted in the presence of a catalyst and a base with an unsaturated $C_{1-6}$alkyl group that is optionally substituted as described above, to form compounds of the general formula I wherein $R^2$ represents an alkenyl or alkynyl radical, and then optionally fully or partially hydrogenated,

c) a compound of the general formula III wherein X, $R^1$ and $R^{3'}$ have the meanings given above is reacted, after previous formylation, with a dithiol, and optionally then the resulting compounds wherein $R^2$ represents

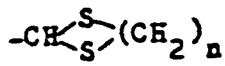

(n = 2 or 3) are converted by reduction to the methyl group or are hydrolyzed to the formyl group which is then optionally reduced to $-CH_2OH$, and the compounds obtained according to processes b and c in which $R^{3'}$ = H are acylated,

and the compounds so obtained are optionally converted into the thioureas and/or the physiologically tolerable acid addition salts are formed.

**4.** Use of the compounds according to claims 1 and 2 for the manufacture of medicaments.

**Revendications**

**1.** Ergolines di-substituées en 1,2 qui répondent à la formule générale I :

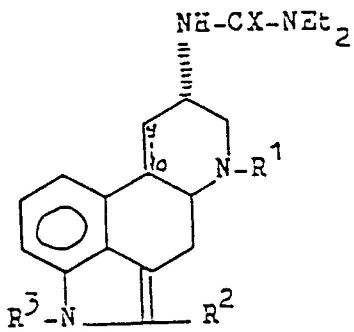

(I)

dans laquelle

X représente un atome d'oxygène ou de soufre,

$R^1$ représente un radical alkyle en $C_1$-$C_6$,

$R^2$ représente :

- un halogène,
- un alkyle en $C_1$-$C_6$, saturé ou insaturé, qui peut éventuellement porter un radical $-OR^4$ ou un radical phényle, pyridyle, thiophényle, furranyle, pyrimidinyle, imidazolyle ou pyrazolyle, lui-même éventuellement porteur d'un alkyle en $C_1$ ou $C_2$, d'un alcoxy en $C_1$ ou $C_2$ ou d'un halogène, le symbole $R^4$ représentant l'hydrogène, un alkyle en $C_1$-$C_6$, un tétrahydropyrannyle ou un cycloalkyle en $C_5$ ou $C_6$,
- un radical $-S-R^5$ dans lequel $R^5$ représente un alkyle en $C_1$-$C_6$ éventuellement porteur d'un phényle, ou un radical phényle, pyridyle, thiophényle, furannyle, pyrimidinyle, imidazolyle ou pyrazolyle, éventuellement porteur d'un alkyle en $C_1$ ou $C_2$, d'un alcoxy en $C_1$ ou $C_2$ ou d'un halogène,
- un radical

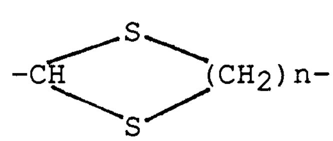

dans lequel n est

égal à 2 ou à 3, ou

- un radical -CHO,

$R^3$ représente un alkyle en $C_1$-$C_6$ ou un acyle en $C_2$-$C_4$ et

$C_9 \cdots C_{10}$

représente une liaison carbone-carbone simple ou double et, lorsque

$C_9 \cdots C_{10}$

représente une liaison carbone-carbone simple, l'atome d'hydrogène de la position 10 a la configuration α,

ainsi que leurs sels d'addition d'acides.

**2.** Composés selon la revendication 1, en l'espèce :

1 la (bromo-2 diméthyl-1,6 ergolinyl-8α)-3 diéthyl-1,1 urée,
2 la (bromo-2 méthyl-1 n-propyl-6 ergolinyl-8α)-3 diéthyl-1,1 urée,
3 la diéthyl-1,1 (propyl-1 iodo-2 méthyl-6 ergolinyl-8α)-3 diéthyl-1,1 urée,
4 la diéthyl-1,1 (méthyl-1 éthynyl-2 méthyl-6 ergolinyl-8α)-3 urée,
5 la diéthyl-1,1 (propyl-1 éthyl-2 méthyl-6 ergolinyl-8α)-3 urée,
6 la diéthyl-1,1 (méthyl-1 phénéthyl-2 méthyl-6 ergolinyl-8α)-3 urée,
7 la diéthyl-1,1 (méthyl-1 phényléthynyl-2 méthyl-6 ergolinyl-8α)-3 urée,
8 la diéthyl-1,1 [méthyl-1 (méthoxy-3 propynyl)-2 méthyl-6 ergolinyl-8α]-3 urée,
9 la diéthyl-1,1 (diméthyl-1,6 méthylthio-2 ergolinyl-8α)-3 urée,
10 la (didéhydro-9,10 diméthyl-1,6 méthylthio-2 ergolinyl-8α)-3 diéthyl-1,1 urée,
11 la (bromo-2 didéhydro-9,10 diméthyl-1,6 ergolinyl-8α)-3 diéthyl-1,1 urée,
12 la diéthyl-1,1 (diméthyl-1,6 phénylthio-2 ergolinyl-8α)-3 urée,
13 la (benzylthio-2 diméthyl-1,6 ergolinyl-8α)-3 diéthyl-1,1 urée,
14 la diéthyl-1,1 (diméthyl-1,6 n-propylthio-2 ergolinyl-8α)-3 urée,
15 la (didéhydro-9,10 diméthyl-1,6 phénylthio-2 ergolinyl-8α)-3 diéthyl-1,1 urée,
16 la diéthyl-1,1 [(dithiolanne-1,3 yl-2)-2 diméthyl-1,6 ergolinyl-8α]-3 diéthyl-1,1 urée,
17 la diéthyl-1,1 (triméthyl-1,2,6 ergolinyl-8α)-3 urée,
18 la diéthyl-1,1 (méthyl-6 formyl-2 ergolinyl-8α)-3 urée,
19 la diéthyl-1,1 (diméthyl-1,6 hydroxyméthyl-2 ergolinyl-8α)-3 urée,
20 la diéthyl-1,1 (iodo-2 acétyl-1 méthyl-6 ergolinyl-8α)-3 urée,
21 la diéthyl-1,1 {acétyl-1 [(tétrahydropyrannyl-2 oxy)-3 propyne-1 yl]-2 méthyl-6 ergolinyl-8α}-3 urée,
22 la diéthyl-1,1 (vinyl-2 propyl-1 méthyl-6 ergolinyl-8α)-3 urée,
23 la diéthyl-1,1 (éthyl-2 propyl-1 méthyl-6 ergolinyl-8α)-3 urée,
24 la (méthyl-6 propyl-1 éthyl-2 ergolinyl-8α)-3 diéthyl-1,1 urée,
25 la diéthyl-1,1 (éthynyl-2 propyl-1 méthyl-6 ergolinyl-8α)-3 urée et
26 la diéthyl-1,1 (propynyl-2 diméthyl-1,6 ergolinyl-8α)-3 urée.

**3.** Procédé pour préparer les composés de formule générale I, procédé caractérisé en ce que :

a) on acyle un composé répondant à la formule générale II :

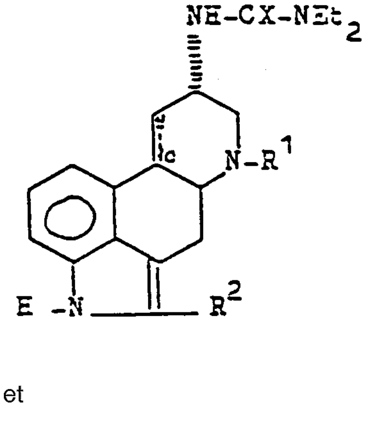

(II)

dans laquelle X, $R^1$, $R^2$ et

C9 . . . C10

ont les significations indiquées ci-dessus, ou, lorsque $R^2$ ne représente ni un radical -S-$R^5$ ni un radical

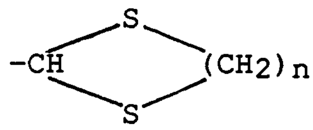

dans lesquels $R^5$ et n ont les significations indiquées ci-dessus, on alkyle un tel composé,
b) on thiométhyle ou on halogène un composé répondant à la formule générale II :

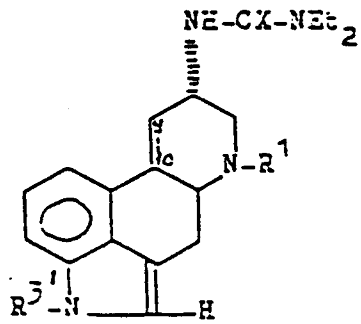

(III)

dans laquelle X et $R^1$ ont les significations indiquées ci-dessus et $R^{3'}$ représente l'hydrogène ou un alkyle en $C_1$-$C_6$, puis éventuellement, lorsque $R^2$ représente un halogène dans le composé de formule générale I ainsi obtenu :

α) on le transforme en la lithium-2 ergoline correspondante et ensuite on fait réagir avec un composé électrophile pour obtenir des composés de formule générale I dans lesquels $R^2$ représente un alkyle en $C_1$-$C_6$ ou un radical -$SR^5$ dans lequel $R^5$ a la signification indiquée plus haut, ou

β) on le fait réagir en présence d'un catalyseur et d'une base avec un radical alkyle en $C_1$-$C_6$ insaturé, éventuellement substitué comme indiqué plus haut, de manière à obtenir des composés de formule générale I dans lesquels $R^2$ représente un radical alcényle ou alcynyle, après quoi on l'hydrogène éventuellement en totalité ou en partie,

c) on fait réagir un composé de formule générale III dans lequel X, $R^1$ et $R^{3'}$ ont les significations indiquées ci-dessus, après l'avoir formylé au préalable, avec un dithiol, puis éventuellement on transforme les composés ainsi obtenus dans lesquels $R^2$ représente un radical

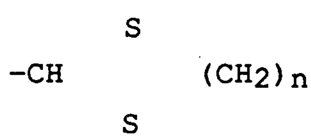

(n = 2 ou 3), par réduction, en le radical méthyle, ou on l'hydrolyse en le radical formyle, après quoi, le cas échéant, on réduit celui-ci en radical -$CH_2OH$, et on acyle les composés obtenus par la méthode b ou la méthode c dans lesquels $R^{3'}$ représente l'hydrogène,

et on transforme éventuellement les composés ainsi obtenus en les thio-urées et/ou on forme les sels d'addition d'acides acceptables du point de vue physiologique.

**4.** Application des composés selon l'une des revendications 1 et 2 à la fabrication de médicaments.